# FASCICULE DE BREVET EUROPEEN

(11) **EP 1 788 943 B1**
(45) Date de publication et mention de la délivrance du brevet: **11.02.2009**
(21) Numéro de dépôt: 05759064.8
(22) Date de dépôt: 08.07.2005
(51) Int. Cl.: A61B 5/113

(54) **DISPOSITIF D'ALARME EN PREVENTION DE LA MORT SUBITE DU NOURRISSON**
ALARMVORRICHTUNG ZUR PRÄVENTION VON PLÖTZLICHEM KINDSTOD
ALARM DEVICE FOR PREVENTING COT DEATH

(30) Priorité: 09.07.2004 FR 0407676
(43) Date de publication de la demande: 30.05.2007
(73) Titulaire: Hartmann, Thierry, 91470 Les Molieres (FR)
(72) Inventeur: Hartmann, Thierry, 91470 Les Molieres (FR)
(74) Mandataire: Thibon-Littaye, Annick
(86) Numéro de dépôt international: PCT/IB2005/001940
(87) Numéro de publication internationale: WO 2006/008604

(56) Documents cités:
- US-A- 5 774 055
- US-A- 5 914 660
- US-A- 6 011 477
- US-A- 6 057 767

## Description

La présente invention concerne un dispositif de prévention de la mort subite du nourrisson, destiné à avertir une personne en charge de la surveillance d'un enfant en bas âge que ce dernier se retourne sur le ventre pendant son sommeil ou pendant une période de repos où il est en position alitée.

Des études récentes ont montré que les enfants en bas âge couchés sur le ventre étaient davantage frappés par le syndrome de la mort subite du nourrisson que ceux couchés sur le dos. Il est donc recommandé de toujours coucher l'enfant sur le dos et de veiller constamment à ce que l'enfant ne se retourne pas de lui-même sur le ventre. Toutefois, cette surveillance constante est quasiment impossible à mettre en pratique, surtout la nuit quand les personnes en charge de la surveillance de l'enfant doivent dormir au même titre que l'enfant à surveiller.

L'objectif de l'invention est, tout en laissant à l'enfant sa liberté de mouvement, de mettre à la disposition des personnes en charge de sa surveillance des moyens qui les avertissent quand l'enfant se retourne de la position dorsale à la position ventrale, y compris quand elles sont à distance de l'enfant et, le cas échéant, en les réveillant quand elles sont endormies.

Les documents US 6 057 767, US 5 914 660 et US 601 1477 montrent des dispositifs faisant partie de l'état de la technique le plus proche.

Le dispositif de prévention de la mort subite du nourrisson suivant l'invention se compose de divers moyens matériels qui se répartissent avantageusement en deux unités distinctes, séparées ou du moins séparables l'une de l'autre, et qui coopèrent les uns avec les autres pour déceler automatiquement si l'enfant en cours de surveillance s'est écarté de la position dorsale suffisamment pour qu'il risque de se retrouver couché sur le ventre et pour commander alors automatiquement l'émission d'un signal d'alarme informant du danger la personne chargée de cette surveillance. Les moyens en question seront ici définis et décrits dans leurs formes de réalisation préférées, d'où ressortiront les caractéristiques de l'invention qui répondent au mieux aux besoins de la pratique, notamment en termes de conditions et coût de fabrication, de solidité, de commodité d'emploi, de propreté sanitaire, de sécurité, d'efficacité de fonctionnement, en apportant ainsi une solution particulièrement satisfaisante au problème de la prévention de la mort subite du nourrisson.

Conformément aux caractéristiques préférées du dispositif suivant l'invention, il est prévu des moyens de transmission à distance d'un signal de commande d'alarme actif en déclenchement de moyens d'alarme à la portée d'une personne en charge de la surveillance d'un enfant, et ce signal de commande d'alarme est produit sous la commande de moyens détecteurs sensibles à l'orientation dans l'espace d'une plaquette montée dans un support à fixer sur le vêtement de l'enfant à surveiller. C'est avantageusement au niveau du nombril de l'enfant que l'on fixe le support de la plaquette portant les moyens détecteurs, ce de manière que le signal déclenchant les moyens d'alarme soit automatiquement généré quand les moyens détecteurs décèlent qu'il s'est produit une variation d'orientation de la plaquette supérieure à un seuil prédéterminé.

La forme et la constitution de la plaquette n'ont pas ici une importance majeure, pas plus que celles de son support, sauf pour ce dernier à rendre facile pour l'utilisateur de le mettre en place sur l'enfant dans une disposition prédéterminée, prescrite pour sa destination en prévention de la mort subite du nourrisson. Il s'agit là que le dispositif soit au repos, sans effet en production de signal d'alarme, quand l'enfant est couché sur le dos, et que les variations d'orientation auxquelles les moyens détecteurs sont sensibles soient en premier lieu celles d'un plan longitudinal passant par la colonne vertébrale de l'enfant, quand il s'incline d'une valeur qui s'exprime par un angle de variation de position angulaire dans un plan transversal, donc normalement vertical mais aussi perpendiculaire à la colonne vertébrale de l'enfant.

Les moyens détecteurs sont, au plus simple, constitués par un détecteur de position angulaire fonctionnant par référence à un axe vertical, tel que déterminé, en direction et en sens, par la gravité terrestre, ou pesanteur. Le seuil déclenchant l'alarme dans le cas d'une variation d'orientation correspondant à une inclinaison de la plaquette par rotation autour de son axe horizontal longitudinal est avantageusement fixé à une valeur angulaire au moins de l'ordre de 90 degrés par rapport à cette référence d'axe vertical, préférentiellement comprise entre 110 et 150 degrés, mais pouvant aller jusqu'à 180 degrés.

En fonction des moyens détecteurs choisis, on peut avantageusement prévoir en plus une détection de toute variation d'orientation de la plaquette dépassant un seuil prédéterminé par inclinaison en rotation autour d'un axe transversal à la colonne vertébrale de l'enfant, et donc notamment autour de la direction horizontale perpendiculaire à l'axe horizontal précédemment cité. De telles dispositions permettent, en second lieu, de signaler à la personne en charge de l'enfant le cas où celui-ci se mettrait en position assise, ou même debout. L'angle seuil de la position angulaire correspondante est avantageusement fixé à une variation de l'ordre de 90 degrés d'arrière en avant (de la tête de l'enfant vers ses pieds). Le cas échéant, cette seconde signalisation sera d'une nature différente de celle de la première, s'en distinguant par exemple en intensité ou en timbre pour un signal sonore, et la personne en charge de l'enfant pourra alors décider s'il est d'âge à rester assis ou si elle va le recoucher avant qu'il ne risque de basculer sur le ventre.

Les moyens détecteurs sont avantageusement prévus au niveau d'une première unité du dispositif, où ils sont associés à des moyens de fixation sur le vêtement de l'enfant, qui peuvent notamment fonctionner par pincement d'une ceinture de pyjama ou de sa couche. Et c'est au niveau d'une seconde unité, normalement séparée et librement mobile indépendamment de la première, que l'on trouve les moyens d'alarme, de préférence intégrés dans un boîtier d'alarme mobile séparément de la première unité, que la personne en charge de la surveillance de l'enfant peut aisément transporter pour le placer loin de l'enfant, mais pour elle à portée de signal d'alarme.

La transmission du signal de commande d'alarme produit à partir des moyens détecteurs s'effectue depuis un émetteur dudit signal faisant partie de la première unité jusqu'à un récepteur coopérant faisant partie de la seconde par tout moyen connu, mais de préférence en utilisant une voie de transmission à distance sans fil. Le signal est donc avantageusement véhiculé dans l'atmosphère sous forme d'ondes électromagnétiques à des fréquences non audibles par l'homme. L'émetteur est commandé par les moyens détecteurs, le récepteur commande l'apparition de l'information d'alarme. Il s'agit avantageusement de déclencher l'émission d'un signal d'alarme à l'intention de la personne en charge de la surveillance de l'enfant, notamment du type d'un avertissement visuel et/ou sonore. Il est en général souhaitable d'éviter l'effet d'interférences extérieures en équipant le dispositif de l'invention d'un système en soi connu, qui peut être du type à code tournant pour accorder automatiquement l'émetteur et le récepteur sur une même fréquence, ou du type à interrupteur codeur pour accord manuel.

Dans le cadre d'un mode de réalisation préféré en conformité avec l'invention, il est avantageux de prévoir, dans le boîtier d'alarme de la seconde unité du dispositif, un logement de rangement de la première unité qui comporte des moyens pour paralyser le fonctionnement des moyens détecteurs, en les empêchant de commander le déclenchement d'alarme. En réalisation concrète de tels moyens, on peut notamment associer un interrupteur à commande magnétique disposé sur le circuit d'alimentation de l'émetteur du signal de commande à distance avec un champ magnétique créé par aimant dans le logement recevant la première unité dans la seconde. Et là bien entendu, un bobinage électrique serait l'équivalent d'un aimant.

D'autres caractéristiques de la présente invention sont plus en relation avec les besoins d'une réalisation filable, qui garantisse confort, hygiène et sécurité pour l'enfant, ainsi que d'un fonctionnement commode et durablement sûr pour la tranquillité des personnes en ayant la charge. Ainsi en est-il de la caractéristique de l'invention qui consiste à enfermer les éléments de la première unité (celle qui se fixe sur l'enfant) dans un coussin molletonné écartant tout risque de blessure sans gêner pour autant la manipulation des moyens qui permettent de la fixer temporairement sur le vêtement de l'enfant. Ainsi en est-il aussi de la réalisation d'un tel coussin, extérieurement, de manière à constituer un objet de décor vestimentaire ou ludique, présentant par exemple l'apparence d'un animal familier.

Ainsi en est-il encore d'une autre particularité de l'invention, qui consiste à noyer les constituants et circuits électroniques de la première unité dans une masse électriquement isolante et étanche, comme on sait couramment le faire en utilisant des matières plastiques à base de polymères. Outre les avantages de solidité, cela entraîne une commodité de démontage de l'ensemble électronique, que ce soit par exemple pour le remplacer, pour changer la pile, ou pour laver le coussin enveloppe.

L'invention sera maintenant plus complètement décrite dans le cadre de caractéristiques préférées et de leurs avantages, en faisant référence aux figures 1 à 3, qui illustrent un mode de réalisation particulier du dispositif suivant l'invention et parmi lesquelles :
- la figure 1 représente, en vue en coupe, une première des deux unités essentielles constituant le dispositif, celle qui est à attacher sur le vêtement de l'enfant à surveiller ;
- la figure 2 est un schéma illustrant la constitution des moyens détecteurs qu'elle comporte ;
- et la figure 3 illustre un mode de rangement particulièrement avantageux de cette première unité dans une seconde unité du dispositif selon l'invention, qui est, elle, à disposer à proximité d'une personne en charge de la surveillance de l'enfant.

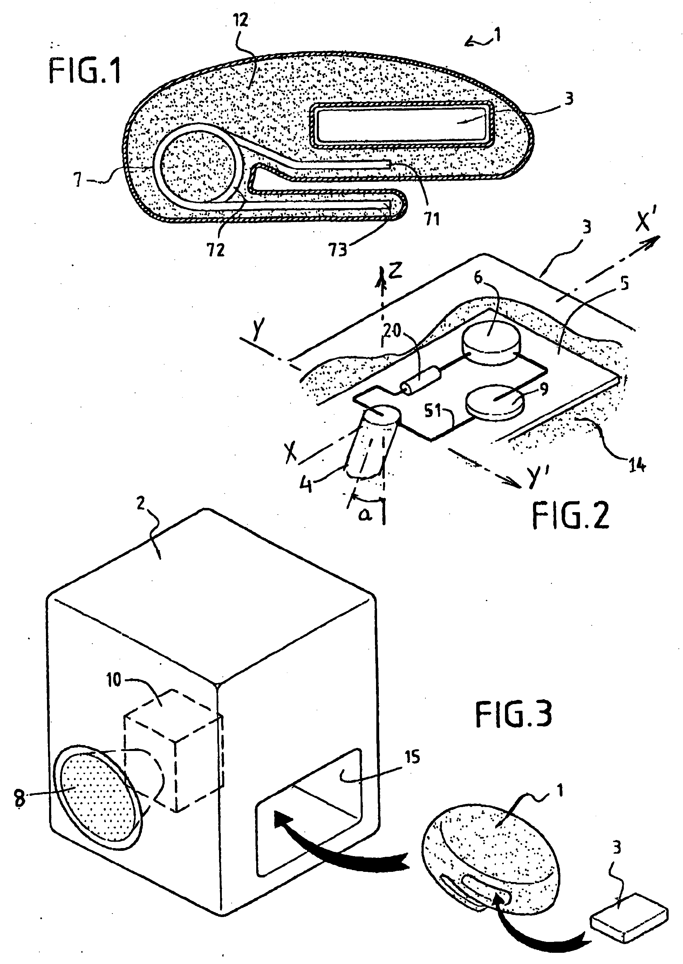

La figure 3 illustre dans son ensemble un mode de réalisation préféré du dispositif d'alarme selon l'invention. Le dispositif est constitué de deux unités, l'une à fixer sur le vêtement de l'enfant, l'autre à disposer au voisinage de la personne en charge de la surveillance de l'enfant.

La première unité du dispositif, portant la référence 1 sur les figures, est réalisée pour être fixée sur le vêtement d'un enfant à surveiller. Cette première unité 1 comporte des moyens 3 réalisés pour commander à distance le déclenchement d'une alarme 10 comprise dans un boîtier d'alarme 2. Le boîtier d'alarme 2 constitue la seconde unité du dispositif selon l'invention. Il est réalisé portatif, de sorte que la personne en charge de la surveillance de l'enfant puisse commodément le placer auprès d'elle.

L'unité 1 contient une plaquette 5, sur laquelle est monté un détecteur de position angulaire 4, qui est sensible à l'orientation de la plaquette dans l'espace et qui fonctionne par référence à la direction verticale définie par la pesanteur en faisant intervenir comme capteur un élément pesant librement mobile dans une chambre fermée. La plaquette 5 supporte un circuit imprimé qui associe le détecteur à un composant émetteur 6 de manière à commander, en fonction de la position de l'élément capteur, l'émission par ce dernier du signal de commande d'alarme. De manière connue en soi, la transmission à distance de ce signal jusqu'à un récepteur coopérant faisant partie de la seconde unité (boîtier d'alarme 2) est effectuée sans utiliser de liaison filaire, donc plus particulièrement par rayonnement hertzien, sous des fréquences inaudibles par l'homme.

La plaquette supporte encore une pile miniature 9 assurant l'alimentation électrique du circuit 51 reliant fonctionnellement le détecteur 4 à l'émetteur 6, et enfin un interrupteur magnétique 20, qui est interposé sur ce circuit pour mettre l'ensemble hors fonctionnement et éviter des déclenchements d'alarme intempestifs.

Dans la plaquette, les différents composants sont noyés dans une masse 14 de résine isolante qui est intégrée, comme on l'a représenté sur la figure 1, dans un coussin protecteur 12. Ce coussin, conçu largement molletonné pour écarter tout risque de gêne ou blessure pour l'enfant, enveloppe aussi une pince 7 servant de moyens de fixation de l'unité 1 sur le vêtement de l'enfant.

Cette pince a été illustrée sur la figure par un ressort 72 rappelant élastiquement deux griffes 71 et 73 entre lesquelles on glisse, par exemple, la ceinture de la couche de l'enfant. Dans la pratique, on préférera en général utiliser une pince de sécurité de l'un quelconque des modèles existant dans le commerce. Dans tous les cas, on prévoit que dans le coussin, le logement de la plaquette 5 soit fixe par rapport à la griffe fixe d'une pince de ce type (ou tout moyen d'attache équivalent). Ceci permet d'imposer une position correcte pour le dispositif en fonctionnement sur l'enfant, en déterminant par exemple, dans le cas décrit, que la plaquette soit disposée dans un plan horizontal quand l'enfant est convenablement couché sur le dos. Le détecteur 4 étant fixe sur la plaquette, la référence verticale est ainsi également déterminée.

L'unité fixe 2 se plaçant au voisinage de la personne en charge de l'enfant est très schématiquement illustrée sur la figure 3 par un boîtier enfermant les circuits électroniques du dispositif d'alarme 10, lesquels comportent un récepteur des signaux de télécommande en provenance de la première unité qui agit en commande de l'émission du signal d'alarme. Ce dernier est ici supposé être un signal sonore, diffusé par un haut-parleur 8. Le cas échéant, on le complétera par un signal visuel.

On observe aussi de la figure 3 que le boîtier d'alarme ménage un logement 15 pour le rangement de l'unité 1 quand celle-ci n'est pas utilisée. Il règne dans ce logement un champ magnétique qui commande l'interrupteur 20 en ouverture permanente du circuit 51. Quand le coussin de l'unité 1 est en place dans son logement, le fonctionnement du système est dès lors paralysé, et quelle que soit l'orientation que prend la plaquette, l'alarme ne peut se déclencher. Le boîtier d'alarme 2 peut facilement être réalisé de manière à être aisément manipulable pour servir à transporter le coussin 12 jusqu'à le mettre en place sur l'enfant quand celui-ci est déjà couché.

On se référera maintenant à la figure 2 pour exposer le fonctionnement du dispositif. Sur cette figure, on a représenté un référentiel trirectangle d'axe Z vertical. Dans le plan horizontal XY on admet que l'axe XX' est orienté le long de la colonne vertébrale de l'enfant, en allant de ses pieds vers sa tête. L'axe YY' est alors horizontal dans la direction transversale, perpendiculaire à la colonne vertébrale de l'enfant, de sa droite à sa gauche.

Le dispositif est conçu pour déclencher l'alarme quand l'enfant se tourne soit vers la droite, soit vers la gauche, jusqu'à se coucher sur le côté, avec même une tendance à passer en position ventrale. Cela s'obtient, conformément à l'un des modes de mise en oeuvre préférés de l'invention, en utilisant comme détecteur un modèle détecteur d'assiette du commerce, essentiellement constitué par une bille en matériau électriquement conducteur mobile dans un logement formant une calotte sphérique à revêtement conducteur, qui est sensible dans toutes les directions aux variations angulaires dépassant un seuil de 120 degrés.

Ce détecteur est fixe dans la plaquette 5. Il y est encastré dans une cavité formée dans un plan vertical longitudinal, qui se trouve être le plan vertical de symétrie ZX contenant la colonne vertébrale de l'enfant quand l'unité 1 du dispositif de l'invention est attachée au niveau du nombril de l'enfant en position correctement orientée. Dès lors, on comprend aisément que quand l'enfant se tourne sur un côté, vers la droite ou vers la gauche indifféremment, il n'est autorisé à le faire que tant que le détecteur s'incline sur la verticale d'un angle restant, dans le plan transversal ZY, en-deça de la valeur seuil de 120 degrés, alors qu'au-delà, la bille du détecteur ferme le circuit d'alimentation de l'émetteur du signal de commande de l'alarme et l'alarme se déclenche.

Dans le plan longitudinal de symétrie du détecteur, la cavité qui le reçoit est inclinée vers le bas vers l'arrière, c'est-à-dire de la tête vers les pieds de l'enfant. L'angle d'inclinaison ("a" sur la figure 2) est de l'ordre de 30 degrés, de sorte que dans ce plan, l'alarme se déclenche dès que l'angle d'inclinaison sur la verticale atteint 90 degrés. Il en résulte que quand l'enfant se met en position assise, l'alarme se déclenche sans attendre qu'il se penche vers l'avant jusqu'à atteindre l'angle de 120 degrés.

L'invention prévoit une autre forme de réalisation avantageuse du détecteur de position angulaire, qui a l'intérêt par rapport à celui de la figure 2 d'être plus simple et moins coûteux, quand on peut se contenter d'une moins grande précision dans les seuils de détection.

Suivant cette variante, non illustrée sur les figures, l'élément sensible du détecteur est constitué par la pile électrique d'alimentation du circuit 51. Pour cela, la pile est montée mobile dans un logement qui se dispose vertical parallèlement à l'axe longitudinal XX' de la plaquette. La pile s'y déplace sous l'effet de la gravité entre un fond du logement où elle reste isolée électriquement et un fond opposé qui est pourvu de contacts fermant le circuit électrique 51. Quand l'enfant se retourne vers la position ventrale en passant par le côté droit ou par le côté gauche, la pile tombe au fond de son logement et ferme le circuit électrique, ce qui déclenche l'alarme. En général, il suffit pour cela que l'inclinaison du logement ait nettement dépassé l'horizontale avec donc une variation de 90 degrés, sans qu'il soit besoin d'atteindre les 180 degrés.

En autre variante, le dispositif selon l'invention peut être construit avec une pile d'alimentation des circuits électriques de la première unité rendue amovible, et aussi aisément remplaçable (ou rechargeable dans le cas d'une pile batterie) quand son niveau de charge devient insuffisant pour garantir le bon fonctionnement de la détection du risque de mort subite du nourrisson.

Par ailleurs, on n'a pas cherché à allonger la description en y mentionnant des détails de réalisation techniques qui sont à la porté de l'homme de l'art en la matière. Ainsi en est-il, par exemple, des moyens de test qui viennent avantageusement compléter les circuits électroniques du détecteur, pour informer l'utilisateur d'une défaillance de l'alimentation électrique. Quand l'utilisateur est prévenu à temps de la survenue d'une défaillance, existante ou prévisible, il lui suffit de changer la pile si celle-ci est amovible. Mais l'indication d'une défaillance, existante ou potentielle, est également fort utile dans le cas où la pile est intégrée avec les autres composants électroniques dans une matrice d'enrobage isolante, sous la forme d'un ensemble jetable.

Il ressort clairement de ce qui précède que l'invention n'est pas limitée aux modes de mise en oeuvre qui ont été spécifiquement décrits et représentés sur les figures.

En particulier, le détecteur a été illustré par des réalisations suivant lesquelles l'élément sensible est une masse mobile. En solution équivalente, on peut utiliser un détecteur dont le fonctionnement est basé sur les variations d'une résistance électrique en fonction de l'orientation de la plaquette. Un tel détecteur peut, par exemple, être associé à un signal d'alarme visuel, produit par des diodes électroluminescentes, qui s'allument en un nombre fonction de l'importance de l'angle d'inclinaison détecté.

## Revendications

1. Dispositif de prévention de la mort subite du nourrisson, comportant deux unités mobiles indépendanmment l'une de l'autre, des moyens détecteurs (4) sensibles à l'orientation dans l'espace d'une plaquette (5) montée dans un support (1) prévus au niveau d'une première desdites unités qui se fixe dans une disposition prédéterminée sur le vêtement de l'enfant à surveiller, des moyens de transmission à distance d'un signal de commande d'alarme qui est produit sous la commande desdits moyens détecteurs (4) et qui est actif en déclenchement de moyens d'alarme (10) intégrés dans la seconde desdites unités, de manière à déclencher les moyens d'alarme (10) en cas de détection d'une variation d'orientation de ladite plaquette (5) supérieure à un seuil prédéterminé, **caractérisé par** des moyens pour paralyser le fonctionnement desdits moyens détecteurs en production dudit signal de commande d'alarme quand ladite première unité est dans une position de rangement au niveau de ladite deuxième unité.

2. Dispositif selon la revendication 1, **caractérisé en ce que** lesdits moyens détecteurs sont constitués par un détecteur (4) de position angulaire fonctionnant sous l'effet de la pesanteur par référence à un axe vertical et **en ce que** ledit support (1) est conformé pour faciliter sa mise en place par fixation sur le vêtement de l'enfant dans une disposition où la position angulaire détectée est au moins celle de la plaquette (5) dans ses variations d'orientation en inclinaison sur un plan longitudinal passant par la colonne vertébrale de l'enfant.

3. Dispositif selon l'une quelconque des revendications 1 ou 2, **caractérisé en ce que** lesdits moyens détecteurs (4) sont associés au niveau de ladite première unité (1) du dispositif à des moyens de fixation (7) sur le vêtement d'un enfant à surveiller dans ladite disposition prédéterminée, avantageusement au niveau de son nombril, cependant que ladite seconde unité intégrant lesdits moyens d'alarme (10) est mobile séparément de ladite première unité (1) pour mettre les moyens d'alarme à la portée d'une personne en charge de la surveillance de l'enfant.

4. Dispositif selon la revendication 3, **caractérisé en ce que** lesdits moyens de fixation (7) sur le vêtement de l'enfant fonctionnent par pincement de la ceinture de sa couche.

5. Dispositif selon l'une quelconque des revendication précédentes, **caractérisé en ce que** ladite seconde unité (2) est réalisée sous la forme d'un boîtier présentant un logement de rangement (15) de la première unité (1) dans lequel règne un champ magnétique apte à commander un interrupteur magnétique (20) d'un circuit électrique (51) d'alimentation des moyens détecteurs, de manière à paralyser le fonctionnement desdits moyens détecteurs en production dudit signal de commande d'alarme quand ladit première unité est en place dans ledit logement de rangement.

6. Dispositif selon l'une quelconque des revendication précédentes, **caractérisé en ce que** ladite première unité (1) est enveloppée dans un coussin protecteur (12) vis-à-vis des risques de blessure de l'enfant.

7. Dispositif selon l'une quelconque des revendications 1 à 6, **caractérisé en ce que** ledit signal de commande d'alarme est transmis sans fil, sous la forme d'ondes électromagnétiques, depuis un émetteur (6) faisant partie de ladite première unité (1) qui est commandé par lesdits moyens détecteurs jusqu'à un récepteur qui fait partie de ladite seconde unité (2) et qui déclenche l'émission d'un signal d'alarme à l'intention de la personne en charge de la surveillance de l'enfant, notamment du type d'un avertissement visuel et/ou sonore.

8. Dispositif selon l'une quelconque des revendications précédentes, **caractérisé en ce que** lesdits moyens détecteurs sont constitués et disposés de manière à commander un déclenchement d'alarme quand la variation d'orientation de la plaquette (5) est supérieure à un premier seuil d'au moins 90 degrés par rotation autour d'un axe longitudinal (XX') se plaçant parallèlement à la colonne vertébrale de l'enfant.

9. Dispositif selon la revendication 8, **caractérisé en ce que** ledit premier seuil est égal à un angle compris entre 110 et 150 degrés.

10. Dispositif suivant la revendication 8 ou 9, **caractérisé en que** lesdits moyens détecteurs sont constitués et disposés de manière à commander un déclenchement d'alarme quand la variation d'orientation de la plaquette (5) est supérieure à un second seuil, limité à environ 90 degrés, par rotation autour d'un axe transversal (YY') se plaçant perpendiculairement à la colonne vertébrale de l'enfant.

## Claims

1. A cot death prevention device, comprising two units that are movable independently of one another, detector means (4) responsive to the orientation in space of a plate (5) mounted in a support (1) provided in a first one of said units that attaches in a predetermined arrangement to the clothing of the infant to be supervised, means of remote transmission for an alarm control signal that is produced under the control of said detector means (4) and that triggers alarm means (10) incorporated in the second of said units for triggering said alarm means (10) in the event of detection of an orientation variation of said plate that is greater than a predetermined threshold, **characterized by** means to hinder the operation of said detector means in producing said alarm control signal when said first unit is in a position of storage in said second unit.

2. A device according to Claim 1, **characterized in that** said detector means comprise an angular position detector (4) functioning under the effect of gravity in relation to a vertical axis, and **in that** said support (1) is so shaped to facilitate its positioning by attachment to the infant's clothing in an arrangement wherein the angular position detected comprises at least that of the plate (5) in its orientation variations in tilt with respect to a longitudinal plane through the infant's vertebral column.

3. A device according to claim 1 or 2, **characterized in that** said detector means are associated in said first unit (1) of the device with means (7) of attachment to the clothing of an infant to be supervised in said predetermined arrangement, advantageously in the area of its navel, while said second unit incorporating said alarm means (10) is movable separately from said first unit (1) to put the alarm means within the reach of a person in charge of the infant's supervision.

4. A device according to claim 3, **characterized in that** said means of attachment to the infant's clothing (7) function by pinching the belt of a nappy it wears.

5. A device according to any of the preceding claims, **characterized in that** said second unit (2) is made in the form of a case having a storage space (15) for the first unit (1) in which a magnetic field is adapted to control a magnet switch (20) of an electrical circuit (51) for supplying the detector means, so as to hinder the functioning of said detector means producing said alarm control signal when said first unit is positioned in said storage space.

6. A device according to any of the preceding claims, **characterized in that** said first unit (1) is enveloped in a cushion (12) protecting the infant against risks of injury.

7. A device according to any of claims 1 to 6, **characterized in that** said alarm control signal is transmitted wirelessly, in the form of electromagnetic waves, from a transmitter (6) integral with said first unit (1) that is controlled by said detector means to a receiver that is integral with said second unit (2) and that triggers the transmission of an alarm signal towards a person in charge of the infant's supervision, said signal being of the visual and/or sound alert type.

8. A device according to any of the preceding claims, **characterized in that** said detector means are made and arranged for controlling triggering the alarm when the orientation variation of the plate (5) exceeds a first threshold of at least 90 degrees by rotation around a longitudinal axis (XX') that is parallel to the infant's backbone.

9. A device according to claim 8, **characterized in that** said first threshold is equal to an angle of from 110 to 150 degrees.

10. A device according to claim 8 or 9, **characterized in that** said detector means are made and arranged for controlling triggering the alarm when the orientation variation of the plate (5) exceeds a second threshold, limited to approximately 90 degrees, by rotation around a transverse axis (YY') perpendicular to the infant's backbone.

## Patentansprüche

1. Alarmvorrichtung zur Prävention von plötzlichem Kindstod, bestehend aus zwei unabhängig voneinander frei beweglichen Einheiten, aus Erkennungsmitteln (4), die in einer ersten (1) der besagten Einheiten angeordnet sind und auf die räumliche Ausrichtung einer ebenfalls in dieser ersten Einheit (1) eingebauten Scheibe (5) ansprechen, wobei die Einheit (1) in vorbestimmter Weise an der Kleidung des zu überwachenden Kindes befestigt wird, weiterhin bestehend aus Mitteln zur Fernübertragung eines Alarmsteuersignals, welches durch das Ansprechen der besagten Erkennungsmittel (4) erzeugt wird und auslösungsaktiv auf in der zweiten Einheit eingebaute Alarmgeber (10) wirkt, so dass diese ausgelöst werden, wenn eine einen vorbestimmten Grenzwert überschreitende Positionsänderung der Scheibe (5) erkannt wird, **dadurch gekennzeichnet, dass** Mittel vorhanden sind, die den Betrieb der besagten, das Alarmsteuersignal erzeugenden Erkennungsmittel blockieren, wenn die erste Einheit sich in Verstauposition im Bereich der zweiten Einheit befindet.

2. Vorrichtung nach Anspruch 1, **dadurch gekennzeichnet, dass** die Erkennungsmittel aus einem Winkeldetektor (4) bestehen, der schwerkraftsbedingt bezüglich einer vertikalen Achse reagiert, und dass die Trägereinheit (1) so ausgestaltet ist, dass sie durch Befestigung an der Kleidung des Kindes leicht in einer Lage angeordnet werden kann, in der die erkannte Winkelposition zumindest gleich der der Scheibe (5) in ihren wechselnden Schräglagen gegenüber einer durch die Wirbelsäule des Kindes führenden Längsebene ist.

3. Vorrichtung nach einem der Ansprüche 1 oder 2, **dadurch gekennzeichnet, dass** die Erkennungsmittel (4) an der ersten Einheit (1) der Vorrichtung Befestigungsmitteln (7) zugeordnet sind, mit denen diese erste Einheit an der Kleidung eines in der vorbestimmten Lage zu überwachenden Kindes befestigt wird, vorzugsweise im Bereich des Bauchnabels, während die zweite, die Alarmmittel (10) umfassende Einheit unabhängig von der ersten beweglich ist, sodass die Alarmmittel in Reichweite einer mit der Überwachung des Kindes betrauten Person gebracht werden können.

4. Vorrichtung nach Anspruch 3, **dadurch gekennzeichnet, dass** die Mittel (7) zur Befestigung der ersten Einheit (1) an der Kleidung des Kindes durch Anklammern dieser Mittel am Gürtelteil der Windel wirken.

5. Vorrichtung nach einem der vorhergehenden Ansprüche, **dadurch gekennzeichnet, dass** die zweite Einheit (2) als Gehäuse ausgebildet ist, in dem ein Verstauraum (15) für die erste Einheit (1) vorgesehen ist, wobei in dem Verstauraum (15) ein Magnetfeld herrscht, das den Magnetschalter (20) eines elektrischen Stromkreises (51) zur Versorgung der Erkennungsmittel so steuern kann, dass der Betrieb der Erkennungsmittel zur Erzeugung des besagten Alarmsteuersignals blockiert ist, wenn die erste Einheit in dem Stauraum abgelegt ist.

6. Vorrichtung nach einem der vorhergehenden Ansprüche, **dadurch gekennzeichnet, dass** die erste Einheit (1) in ein Schutzkissen (12) eingehüllt ist, um jegliche Verletzung des Kindes zu vermeiden.

7. Vorrichtung nach einem der Ansprüche 1 bis 6, **dadurch gekennzeichnet, dass** das besagte Alarmsteuersignal drahtlos in Form von elektromagnetischen Wellen übertragen wird, ausgehend von einem in der ersten Einheit (1) eingebauten und über die Erkennungsmittel (4) gesteuerten Sender (6) bis zu einem in der zweiten Einheit (2) integrierten Empfänger, der ein insbesondere visuelles und/oder akustisches Alarmsignal auslöst.

8. Vorrichtung nach einem der vorhergehenden Ansprüche, **dadurch gekennzeichnet, dass** die Erkennungsmittel (4) so ausgelegt und angeordnet sind, dass sie ein Alarmsignal auslösen, wenn die Positionsänderung der Scheibe (5), durch Drehung um eine parallel zur Wirbelsäule des Kindes verlaufende Längsachse (XX'), einen ersten Grenzwert von mindestens 90 Grad überschreitet.

9. Vorrichtung nach Anspruch 8, **dadurch gekennzeichnet, dass** der besagte erste Grenzwert gleich einem Winkel von 110 bis 150 Grad ist.

10. Vorrichtung nach Anspruch 8 oder 9, **dadurch gekennzeichnet, dass** die Erkennungsmittel (4) so ausgelegt und angeordnet sind, dass sie ein Alarmsignal auslösen, wenn die Positionsänderung der Scheibe (5), durch Drehung um eine senkrecht zur Wirbelsäule des Kindes verlaufende Querachse (YY'), einen zweiten Grenzwert von höchsten ca. 90 Grad überschreitet..
